# EUROPEAN PATENT SPECIFICATION

(11) **EP 0 904 701 B1**
(45) Date of publication and mention of the grant of the patent: **01.06.2005**
(21) Application number: 98116181.3
(22) Date of filing: 27.08.1998
(51) Int. Cl.: A23L 1/03, C12N 1/00, A23K 1/00, A23K 1/175, A23L 1/30, A23L 1/304, A61K 35/66

(54) **Inactivated micro-organisms containing minerals, process for their preparation, and their use in food, veterinary and pharmaceutical compositions**
Inaktivierte Mikroorganismen, welche Mineralien enthalten, deren Herstellung und Verwendung in Lebensmitteln, pharmazeutische und zootechnische Zusammensetzungen
Micro-organismes inactivés contenant des minéraux, leur production et utilisation dans des compositions alimentaires, pharmaceutiques ou vétérinaires

(30) Priority: 29.08.1997 IT MI971988
(43) Date of publication of application: 31.03.1999
(73) Proprietor: DOX-AL ITALIA S.p.A., I-20050 Correzzana Milano (IT)
(72) Inventor: Volpato, Ivo, 06070 S. Mariano (Perugia) (IT); Bizzini, Bernard, 46100 Figeac (FR); Veneroni, Flavio, 20050 Correzzana (Milan) (IT)
(74) Representative: Gervasi, Gemma, Dr.

(56) References cited:
- DE-C- 424 658
- FR-A- 748 741
- FR-A- 2 179 528
- US-A- 4 348 483
- DATABASE WPI Section Ch, Week 198730 Derwent Publications Ltd., London, GB; Class D11, AN 1987-208744 XP002230810 & JP 62 134083 A (IWAKI SEIYAKU KK), 17 June 1987 (1987-06-17)
- NIETO J M ET AL: "PREPARATION AND CHARACTERIZATION OF A CHITOSAN-FE(III) COMPLEX" CARBOHYDRATE POLYMERS, APPLIED SCIENCE PUBLISHERS, LTD. BARKING, GB, vol. 18, no. 3, 1992, pages 221-224, XP000275394 ISSN: 0144-8617

## Description

### SCOPE OF INVENTION

The present invention relates to a new process for obtaining inactivated micro-organisms which may be used as vehicles of minerals, possibly in the form of complexes with polysaccharides in human food or feed for animals. Said micro-organisms moreover exert a probiotic activity and are particularly useful as components of premix and animal feed for rearing livestock.

### STATE OF THE ART

The optimization of the nutritional intake of minerals through integration of food is necessary both under normal conditions and in the presence of physiological alterations of the organism due to pathological states. In fact, there is frequently observed an inadequate intake of minerals on account of the presence of numerous factors of inhibition of their absorption, such as the nature of the mineral salt administered or the presence of substances which are able to capture said minerals, forming insoluble complexes with the latter. These factors render the intake of minerals through common food diets insufficient.

Among the minerals of physiological importance, the following should be recalled: **copper,** deficiency of which is implicated in skeletal alterations and alterations of the cardiovascular and nervous systems, as well as in forms of lymphomas and leukaemia; **zinc,** which has the function of activating the immune system and is involved in enzymatic and hormonal activities, as well as in physiological activities of growth and development; **iron,** deficiency of which is generally linked with anaemic states and related illnesses; **selenium,** which is able to prevent cellular damage caused by peroxides and free radicals; **chromium,** which has potential activity in states of stress and physical effort; **iodine,** which is fundamental for the biosynthesis of thyroid hormones; **manganese,** which is an enzymatic activator and essential component of cellular membranes; **calcium,** which is a major constituent of bone tissue and plays important physiological roles in muscle contraction, cardiac function, neurotransmission, etc.; **magnesium,** which is an activator of a wide range of enzymatic systems and is prevalently located in bone tissue; **gold,** which performs a fundamental role in antagonizing rheumatoid disorders; and **platinum,** notoriously known as anti-tumour agent.

From what has been illustrated above, it emerges that the aforesaid minerals are involved in the balancing of important biochemical processes, the alteration of which leads to onset of serious pathological events.

The problem linked to insufficient mineral intake is particularly felt in the field of zootechnics, i.e., in the rearing of livestock, where an appropriate food intake plays a fundamental role in achieving the maximum degree of competitivity, markedly affecting the general state of health of the animal, as well as the quality of the end produce (for example, meat and milk). In animal rearing, there is a general tendency to resort to an indiscriminate increase in the concentration of minerals added to the feed, in the hope of increasing the amount that can be absorbed. However, such increases frequently fail to have the effect of raising the intake of minerals; on the contrary, in addition to increasing costs, they lead to the onset of toxic phenomena. Furthermore, a poor homogenization of these elements in the feed may lead to serious side effects, besides contributing in a harmful manner to the rise in contaminating inorganic waste. In the state of the art, the optimization of the intake of minerals, and in particular of rare minerals, is commonly pursued by associating appropriate mineral salts to normal human or animal food. The exogenous supply of minerals may be effected via different administration routes, preferably the oral route, in the form of salts or organic and inorganic derivatives. However, for the majority of the aforesaid minerals, there arise difficulties of administration linked to the poor oral absorption in the case of doses of therapeutic interest and to the possibility of onset of toxic effects, among which that exerted by a prolonged contact with the gastro-intestinal mucous membrane. The above-mentioned minerals and their derivatives frequently compete with one another for intestinal absorption and present different levels of bio-availability, absorption and toxicity. In addition, the concentrations in the blood and body tissues required for the individual minerals vary considerably from one mineral to another, involving small quantities, for example in the case of the so-called "rare" minerals Cu, Se, Cr, Au, Pt, Mn, Mo, Zn, Cd, V and Li, and significantly larger quantities for Fe, Ca, Mg and K.

To overcome the aforementioned problems, in the state of the art various derivatives have been studied that are able to increase the level of absorption of the minerals and/or to improve their characteristics of bio-availability. There are known to the state of the art derivatives of the aforementioned minerals with substances of various chemical nature, such as surfactants, inorganic salts, amino-acids or proteins, vitamins, and carbohydrates. Very often, however, the use of such ligands has proved incompatible with therapeutic uses on account of the unacceptable levels of toxicity of the ligands themselves.

More recently, glucans have been employed as chelating agents, since they possess good complexing capacities related to the presence of primary amine groups along the polysaccharide chains. These ligands may form, with the various metals, both intramolecular and intermolecular complexes which may vary according to the pH conditions, concentrations, temperatures, etc. (as regards iron, see for example J.M. Nieto *et al*., *Carbohydrate Polymers*, 18: 221-224, 1992).

However, also these ligands are accompanied by side effects, which prevent their widespread therapeutic use; in fact, they do not allow any accurate control of absorption. This impossibility of administering reproducible and controlled doses of minerals may principally be due to the fact that, during their passage through the gastro-intestinal tract, there frequently occurs the exchange of the minerals with other cations that are present. In addition, the stability of the chelates formed by these ligands may be impaired by substances that are present, such as lectins, amino-acids, fatty acids, etc.

FR-A-2 179 528 teaches a method to enable penetration, fixation and/or absorption of substances, eg Cl₂Mg, by micro-organisms, eg yeast, by treating the living biological mass with a plasmolytic agent, eg propylene glycol, after which said substances are added and they penetrate into the cells of the biological mass.

The need is therefore felt for the possibility of administering such oligo-elements in a way which is at the same time reproducible, convenient and economic, guaranteeing optimization of the dispersion of the minerals in the feed and preventing harmful accumulations and unhomogeneous administrations of the same to livestock animals. This would result, in fact, in advantages of an economic nature (reduction of quantities to be administered per day), as well as of an environmental nature (reduction of waste in the faeces), and of a pharmacotoxicological nature.

### SUMMARY

Object of the present invention is a process for producing inactivated micro-organisms containing minerals in the form of complexes with polysaccharides, which comprises the following steps:
1) extraction of the endocellular mass from the cells of an appropriate micro-organism by means of hyperosmotic treatment, and separation of the extracted endocellular mass by centrifugation or filtration;
2) possible inactivation of the micro-organism obtained in step (1) by chemical or physical means, leaving the external membrane of the micro-organism unaltered;
3) intracellular insertion of one or more minerals in the inactivated micro-organism obtained in step (1) or (2) by means of hypo-osmotic treatment, by treating the said micro-organism in a hypotonic suspension containing the endogenous material that has been extracted in step (1), to which are added minerals in the form of complexes with polysaccharides, or else in a suitable hypotonic aqueous suspension containing said minerals in the form of complexes with polysaccharides.

The present invention moreover regards the inactivated micro-organisms containing minerals, obtained with the process described above, the compositions containing them, possibly in association with appropriate food, excipients and/or diluents, as well as their use in food, both in the human field and for veterinary purposes, and in particular in zootechnics as components of animal feed and premix for livestock.

### DETAILED DESCRIPTION OF THE INVENTION

The characteristics and advantages of the process for the production of inactivated micro-organisms enriched with minerals, of the micro-organisms thus obtained, of the compositions containing them, and their use in the human or veterinary food sector, according to the present invention, will be more fully illustrated in the course of the following detailed description.

The present invention enables the administration of minerals in the form of complexes with polysaccharides, incorporated inside the cellular walls of micro-organisms that have been inactivated, i.e., "killed", which are able to protect the activity thereof.

Said minerals are preferably selected from the group consisting of Cu, Zn, Fe, Se, Cr, Mn, Ca, Mg, Mo, Au, Pt, Cd, V, Li, and K.

The introduction of the minerals into the inactivated micro-organisms according to the present invention prevents the latter, during their passage through the digestive tract, from coming into contact with substances that are able to form non-absorbable insoluble complexes. Furthermore, this vehicling makes available the minerals themselves for the complexing with the carbohydrates and the transport proteins present in the absorption site and in the cellular membrane of the micro-organism.

The above-mentioned minerals may be inserted into the inactivated micro-organisms also in the form of complexes with polysaccharides. These polysaccharides, which are of bacterial, algal, animal, and vegetal origin, are preferably anionic and may be selected from the group consisting of alginates, xanthans, mannans, dermatan sulphates, chondroitin sulphates, dextrans and their fragments.

The aforesaid polysaccharides, before complexing with the appropriate minerals, are preferably depolymerized by means of enzymatic treatment, for example as described by Y. Yonemoto *et al*. (*Journal of Fermentation and Bioengineering*, 75(1):68-70, 1993), obtaining oligosaccharides with different ranges of molecular weight (MW), preferably between 2,500 and 5,000 daltons.

The process of complexing for the formation of the chelates between polysaccharides, preferably oligosaccharides, and the minerals in the cationic form takes place according to methodologies that are already known to the present state of the art. Preferably, to an aqueous solution of appropriate oligosaccharides in the quantity of 3-10% by weight is added the mineral to be chelated in excess, preferably in the form of a salt (chloride, citrate, fumarate, ascorbate, etc.), and the reaction mixture is left to react at room temperature for 10-20 hours. The reaction pH varies according to the cation to be complexed. This complexing may be conducted in the presence of a suitable reducing agent, such as ascorbic acid in concentrations of 0.5-5% by weight. After the excess of salts has been eliminated by means of gel filtration, for example on Sephadex® G10, the mixture is vacuum-evaporated at the temperature of 40-50°C to obtain the chelated ion in concentrations of 1.5-4% by weight. The determination of the exact concentration of chelated ion may be carried out using specific spectrophotometric methods commonly employed in the state of the art.

Finally, the aforesaid salts may be inserted in the micro-organisms at the same time as the aforesaid polysaccharides (without having first performed complexation), so that they will become available in the absorption site directly as chelates.

In the case in which the mineral is inserted into the micro-organism in the form of a salt, the polysaccharide for the formation of the possible chelate may be supplied by the cellular wall of the micro-organism itself, which lyses during the digestive process. The micro-organisms used in the process according to the present invention must possess good characteristics of resistance to chemical and physical-chemical stress, being able to act as vehicles for the minerals incorporated at the endocellular level. These micro-organisms may be:
(i) micro-organisms not producers of specific polysaccharides, used after inactivation as vehicles of one or more minerals, which may be inserted into the micro-organisms themselves in the form of salts and/or of complexes with specific polysaccharides;
(ii) micro-organisms that are producers of one or more specific polysaccharides.

In both cases, the micro-organisms may be used for transporting minerals in the form of mineral salts or complexes with polysaccharides, either produced or not produced by the micro-organisms themselves.

In **case (i),** the strains of micro-organisms must have appropriate characteristics of resistance to chemical and physical-chemical agents, as they must be able to reach the intestine structurally intact.

Amongst the known strains available on the market, the *Saccharomyces cerevisiae* is preferred, this being able to reach intact the site in which the intestinal digestive process takes place. Said micro-organism moreover performs an advantageous probiotic activity, as described in the European patent No. EP 0 111 202.

Before the emptying-out step, the micro-organisms may be grown in appropriate fermenters, according to the methods and conditions known to the state of the art, the micro-organic mass being separated from the culture medium, at the end of the growth step, by means of filtration or centrifugation.

In **case (ii),** it is possible to use micro-organisms that are known for the production of specific polysaccharide substances, which are readily available on the market. Said micro-organisms may be producers of endopolysaccharides, which remain inside the cell, of exopolysaccharides, which are released in the growth medium, or of structural polysaccharides, which serve as components of the micro-organic walls.

For the production of alginates, *Azotobacter vinelandii* and *Pseudomonas aeruginosa* are preferably used; for the production of xanthans, *Xanthomonas campestris* is preferably used; finally, for the production of phosphomannans, *Hansenula capsulata* is preferably used.

Before the emptying-out step (1) of the process of the invention, the selection of appropriate micro-organisms is obtained, according to methods known in the state of the art, for the development of the microflora in nutrient culture and subsequent selection of the colonies on the basis of their morphological and taxonomic characteristics. The characterization of the isolated strains is carried out according to methods commonly used, for example by analysis of Gram's stain, metabolic behaviour, production of characteristic chemical products, and nutritive characteristics.

The fermentative growth of the micro-organism is obtained, with production of saccharidic biopolymers, according to the batch or continuous techniques commonly used in the state of the art, for example in the operating conditions referred to in *Biochemical Engineering and Biotechnology Handbook*, B. Atkinson and F. Mavituna Eds., 1991, MacMillan Publ., Chapter 6.14, pages 350-353.

The micro-organisms are separated from the growth medium by filtration or centrifugation, recovering the supernatant in the case where the saccharidic biopolymers of interest are exopolysaccharides (i.e., released in the growth medium). The supernatant is then concentrated, and the polysaccharides of interest may be recovered by precipitation, for example with 95% isopropanol, in the volumetric ratio 2:1 with respect to the mixture, as described by W.P. Chen *et al*. (*Applied and Environmental Microbiology*, 49(3):543-546, 1985). The precipitated polysaccharide, after centrifugation and washing with water to eliminate the residual alcohol, may be subsequently used in the re-incorporation step (3). The aforesaid polysaccharides may alternatively be precipitated with ethanol (3 volumes) in the presence of 1% potassium acetate. The concentration of polysaccharides in the precipitate may readily be determined using standard methods, for example as described by K. Ostgaard (*Carbohydrate Polymers*, 19:51-59, 1992). The polysaccharides thus obtained, before the re-incorporation step (3), are preferably subjected to depolymerization for the production of oligosaccharides, for example as described by Y. Yonemoto *et al*. (*Journal of Fermentation and Bioengineering*, 75(1):68-70, 1993), by means of enzymatic treatment (for example, using alginate lyase in the case of alginates and using chondroitinase in the case of dermatan sulphate), for a sufficiently long time to produce oligosaccharides of molecular weight of 2,500-5,000 daltons. The process of lysis may advantageously be controlled by employing turbidimetric methods, for example as described by M. Kitamikado *et al*. (*Applied and Environmental Microbiology*, 56(9):2939-2940, 1990) or electrophoretic methods, for example as described by R. Cappelletti *et al*. (*Analytical Biochemistry*, 99:311-315, 1979). At the end of lysis, the fractions of oligosaccharides may then be recovered by means of precipitation with ethanol. The control of the quantity of oligosaccharides and of their MW may be performed by means of gel filtration chromatography, for example on Sephadex® G25. The concentration of oligosaccharides in the eluted fraction may be determined by addition of Alcian Blu reagent and subsequent spectrophotometric analysis, as described by A.J. Pesce *et al*. (*Methods in Clinical Chemistry*, 1987, Mosby Publ., pages 189-200). By operating with reference to standard solutions with known molecular weight, finally it is possible to establish the molecular weight of the various fractions recovered.

The aforementioned strains may moreover be subjected to a process of conditioning for the production of specific polysaccharides, with the aim of optimizing the production yields, using methods commonly employed in the state of the art. After the conditioning step, also these micro-organisms undergo a process of fermentation as described above, to produce the desired polysaccharides, before passing on to the emptying-out step (1).

In the emptying-out **step (1),** the endocellular mass is caused to come out of the micro-organic cellular walls by means of the hyperosmotic treatment of "squeezing" of the micro-organisms obtained as described above. In the case where the micro-organisms are previously subjected to fermentation with the purpose of producing the desired polysaccharides, the latter are taken off at the maximum peak of the cellular growth curve, before start of the step of stasis. Step (1) is carried out by suspension of the micro-organic mass in a hypertonic aqueous solution containing:
- NaCl in concentrations of over 0.2-0.3 M;
- sodium citrate in concentrations of over 0.05 M;
- an appropriate antibacterial agent, preferably thimerosal (in particular Merthiolate®, 1997 Sigma catalogue), in suitable concentrations.

Preferably the aforesaid hypertonic solution contains NaCl 1 M, sodium citrate 0.1 M, and thimerosal 0.1 mg/l.

The suspension thus obtained is then kept under agitation at a temperature of between 2 and 8°C, preferably 4°C, for a period of between 2 hours and 4 days, preferably 72 hours, obtaining the squeezing of the micro-organisms as the endocellular content is extruded into the hypertonic medium. The endocellular mass which is caused to come out of the cells may comprise also the exopolysaccharides produced.

The micro-organisms thus emptied out and reduced to their cellular walls alone are smaller than normal, and the membrane pores are enlarged. The micro-organisms are separated from the endocellular mass that has come out in the suspension medium by means of filtration or centrifugation.

The control of the emptying-out of the micro-organic walls may be conveniently carried out by microscopic evaluation of the morphology of the walls themselves, which must appear smaller and wrinkled.

Once the emptying-out step is terminated, the suspension medium (i.e., the hypertonic buffer and the endocellular content that has been extracted) may be separated from the emptied micro-organisms by means of centrifugation at 8,000-15,000 r.p.m., preferably 10,000 r.p.m. In the case of the micro-organisms (ii), the suspension medium is appropriately treated for the recovery of the exopolysaccharides contained therein and for the elimination of the saline hyperconcentration. In particular, this medium may be dialysed against water on a membrane with molecular cut-off at 10,000 daltons for a period of 48 hours, and the polysaccharide content may be isolated and finally used for the preparation of the hypotonic medium used in step (3).

In **step (2),** the micro-organisms which have been emptied of their endocellular mass may be inactivated, i.e., killed, by means of suitable chemical or physical treatment. Such treatment may not be necessary for certain micro-organisms, in so far as the hypertonic treatment of step (1) in many cases already proves sufficient to inactivate the cells. Hence, this step (2) may be carried out after the state of inactivation of the micro-organisms obtained from step (1) has been verified.

Among the chemical methods preferably used for micro-organisms having cellular walls that are easily degradable to heat, treatments using disinfectant substances (with concentrations of 0.05-0.2 mg/l) may be employed for a period of 4-72 hours, and preferably with thimerosal, at a concentration of 0.1 mg/l, for a period of 6-8 hours. At the end of the treatment, the micro-organic mass is recovered by filtration or centrifugation, washing the thimerosal residue with water.

This chemical inactivation may take place also during the previous emptying-out step, by adding the aforementioned disinfectant substances to the hypertonic solution.

Physical treatments preferably comprise exposure to UV rays and thermal inactivation by heating of the suspension obtained from step (1) at a temperature of between 55 and 65°C, preferably 60°C, and finally isolating the inactivated micro-organisms by concentration and filtration.

This treatment may be carried out also at the start of step (3) of intracellular re-integration in hypotonic medium. In this case, the emptied micro-organisms obtained from step (1) are suspended in a part of the hypotonic medium containing the minerals to be re-incorporated, and the mixture thus obtained is then heated up in the conditions referred to above. After cooling to 2-8°C, preferably 4°C, the remaining part of the hypotonic medium is added, and the next step of intracellular re-integration proceeds as described in step (3).

The conditions of inactivation must be such as not to cause alterations in the parietal structure of the micro-organism, and hence must be chosen according to the characteristics of resistance of the micro-organism itself.

The control of the degree of inactivation of the micro-organic cells at the end of step (2) may be carried out using culture tests for the control of viability as follows: a small aliquot of treated micro-organic cells are re-suspended in part of the previously extracted endocellular mass, after dialysis of the same for elimination of the salts and after having re-established isotonicity. When the cells have reacquired their original shape, approximately 1 g or 1 ml of their suspension is inoculated in a selective growth medium (approx. 15-25 ml of nutrient broth), and the cells are left in incubation (at approx. 37°C for bacteria; at approx. 25°C in the case of Mycetes) for a period of 36-48 hours. Since this first step may not be sufficient to restore totally viability to the micro-organism, the sowing may be repeated for further subsequent transplants (preferably 3), using the broth obtained from the first step. Possible processes of growth are controlled by analysing the concentration of the colonies present by means of turbidimetric analysis, or under the microscope (on slides after staining), or else by using colony counters.

In **step (3),** there is the intracellular integration, in the inactivated micro-organic cells, of one or more minerals to be vehicled, by means of treatment in a hypotonic aqueous solution containing said minerals in the form of complexes with polysaccharides, in which the molarity of the ion is preferably between 0.01 and 0.05 M, the molar ratio of conjugation between ion and polysaccharide being preferably between 1:1 and 10:1. The mixture thus obtained is kept under gentle stirring for a period sufficiently long to enable re-incorporation of the material in the cells, ranging between 8 hours and 4 days, more preferably 72 hours, at a temperature of between 2 and 8°C, preferably at 4°C. The concentration of salts and chelates may also be progressively increased, but always remaining in conditions of hypotonicity. The concentration of the minerals in the reaction medium may be controlled periodically via spectrophotometry or by monitoring of the cryoscopic point. The addition of the mineral in the form of complex with polysaccharides is preferably made until the decrease in the concentration of said minerals in the reaction medium is no longer detected.

The aforesaid hypotonic aqueous solution preferably contains, in addition to the above-mentioned minerals, an appropriate antibacterial agent, preferably thimerosal (in particular Merthiolate®, 1997 Sigma catalogue), in suitable concentrations.

Preferably the aforesaid hypotonic solution contains NaCl 0.05 M, sodium citrate 0.005 M, and thimerosal 0.1 mg/l.

During the operation of intracellular re-integration, the biomass and the minerals contained in the hypotonic suspension are absorbed within the inactivated micro-organisms, which re-acquire their original shape. The re-absorption control may be conducted evaluating the quantity of minerals still present in the hypotonic aqueous suspension, using spectrophotometric techniques, or else by means of spectrophotometric analysis of the content of minerals in the micro-organic cells, following on lysis of the same and analysis of the lysate. In this case, preferably an aliquot of cells is suspended in a PBS buffer at pH 7.4, and the cells are sonicated for a period of between 30 seconds and 2 minutes. The cellular lysate is kept under gentle stirring for 30 minutes at 4°C and is then centrifuged at 10,000 r.p.m. for 15 minutes. The quantitative determination of the various minerals may then be performed using standard methods, such as atomic absorption, spectrophotometry, or spectrofluorometry. In the case of cells used only as vehicling agents (case (i)), the inactivated micro-organisms may be immersed in a hypotonic suspension medium containing one or more minerals in the form of salts and/or complexes with polysaccharides.

In the case, instead, where the micro-organisms (ii) are used, the said aqueous suspension may consist of the endocellular mass extracted in step (1) containing the aforesaid minerals complexed with the polysaccharides previously produced by the micro-organism. The suspension medium containing the endocellular mass obtained in step (1) is previously subjected to dialysis up to total elimination of the salt used for the previous hyperosmotic treatment.

In the case where the polysaccharides produced by the micro-organism are exopolysaccharides, the suspension medium may consist either of polysaccharides recovered from the fermentation broths of the previous fermentation step, as described above, or from the endocellular mass squeezed out in step (1). Differently, in the case of endopolysaccharides, only the aforesaid endocellular mass is used.

The micro-organisms obtained from step (3) may finally be separated by means of filtration or centrifugation, washed with a sterile hypotonic solution, and finally re-suspended in small volumes of a sterile hypotonic solution. In this state, the said micro-organisms may be mixed with appropriate integrators or foods, in the preparation of food compositions, in concentrations suitable for obtaining an optimal contribution of minerals in man or animals.

A further object of the invention is constituted by the micro-organisms containing minerals, obtained by the process described above. Such micro-organisms serve as excellent vehicles, at an intracellular level, of the minerals themselves and enable their administration both in food for humans and for veterinary purposes. This vehicling rules out the risk of reduction of the absorption of the minerals themselves, which could be complexed during their passage through the digestive tract.

After intake of the micro-organisms of the invention by humans or animals, through the food or feed, in the step of metabolization at the intestinal level the fragmentation of the cell walls of the micro-organisms leads to the release of the incorporated minerals, possibly complexed with the aforesaid polysaccharides.

The complexing of the minerals with the polysaccharides significantly facilitates the gastro-intestinal absorption of the minerals themselves, as it guarantees a proper degree of ionization of the mineral in the absorption site. It should in fact be remembered that the bio-availability of oligo-elements is markedly conditioned by their degree of ionicity. The presence itself of the polysaccharides as complexing agents is able to influence the affinity between the oligo-elements and the tissues, enhancing the degree of their absorption. Furthermore, the polysaccharides, in addition to advantageously complexing the minerals, thus facilitating their absorption, perform a probiotic function.

The micro-organisms of the invention are particularly useful in zootechnics since they lead to an improvement of the general state of health of the animal, and consequently the quality of the produce to be consumed (milk, eggs, meat, etc.). In particular the micro-organisms of the invention show the following pharmacological effects:
- probiotic function: the non-polymeric fragments of the micro-organism, obtained from the fragmentation which occurs in the animal at the intestinal level, have a nutritive value;
- effects of immunostimulation and normalization of intestinal flora: in fact, certain components of the micro-organic membranes, such as glycoproteins and peptidoglycans, perform a normo-regulating activity on the mucous membrane, promoting its functionality and stimulating the immunoprotective reactions of the animal itself;
- improvement of the growth curves of the animals and consequent reduction in breeding costs.

Finally, the inactivated micro-organisms according to the present invention offer the advantage of enabling homogeneous dispersion of the minerals in the feed, thus avoiding the inconvenient side effects known in the state of the art, which are due to accumulation of minerals in the preparations and to the unhomogeneous administration of optimal, controllable and reproducible quantities of said minerals.

Further advantages are represented by the low production costs achieved, which are compatible with extensive applications, for example in the zootechnic sector, as well as the facility of use in the animal feed industry.

Thus a further subject of the present invention is the use of the aforesaid inactivated micro-organisms containing minerals in the food sector, both for human and for veterinary purposes. Said micro-organisms may be administered in physiological states or pathological conditions connected with deficit of specific minerals.

In the area of human use, the micro-organisms of the invention enable administration of Pt as anti-tumoural agent, Zn in immunoregulation, Au and Cu and/or Zn in inflammatory diseases, Fe in anaemic states, etc.

Insofar as the minerals contained in the inactivated micro-organisms are endowed with a therapeutic activity, the inactivated micro-organisms also work as carriers of pharmaceutically active principles, and can be formulated and used as pharmaceutical compositions.

These compositions may, in addition to said minerals, contain further active principles.

In zootechnics, and preferably in the rearing of livestock, the micro-organisms of the invention enable the administration of minerals in integration of food with the purpose of obtaining an improvement in the general state of health of the animal and an increase in its immune defences. In particular, they enable the administration of Ca and Mg in egg-producing species.

Finally, a further object of the present invention is represented by food compositions, usable in food for both human and veterinary purposes, comprising one or more inactivated micro-organisms enriched with minerals in the form of salts and/or complexes with polysaccharides, as described above, in association with conventional foods, and possibly in the presence of appropriate excipients and/or diluents.

The quantity of micro-organisms to be administered varies according to the animal species treated, the diet followed, the general state of health of the animals, and the conditions of rearing, and is preferably between 0.1 and 20 g/kg of feed for livestock, and from 0.1 to 5 g/day in food for human use.

To provide an illustration of the present invention, the following non-limiting example is given.

### EXAMPLE 1

### Preparation of inactivated Saccharomyces cerevisiae containing calcium, according to the present invention.

Cells of *Saccharomyces cerevisiae*, isolated from by-products of the processing of sugars using ordinary laboratory techniques, were made to grow by fermentation on cane sugar molasses in a 75-m³ fermenter, with aeration of 2 mM 02/l • min, under agitation of 1 vvm, at a temperature of 30°C, with a pH of 4.5-5.5, and a culture density of 40-45 g/l. The cells were then separated by means of centrifugation at 10,000 r.p.m. for 15 minutes to obtain 0.5 g of dry product (corresponding to 15 • 10⁹ cells) per 1 g of original substrate.

1 kg of cells of Saccharomyces *cerevisiae* thus obtained was suspended in 25 l of a hypertonic aqueous solution containing NaCl 1 M, sodium citrate 1 M, and Merthiolate® 0.1 mg/l (1997 Sigma catalogue), and the suspension was kept under gentle stirring for 72 hours, at a temperature of 4°C. The suspension thus obtained was then centrifuged at 10,000 r.p.m. for 15 minutes, the cells were recovered, and their inactivation was verified.

In 25 l of a hypotonic calcium aqueous solution containing CaCl₂ • 2H₂O 0.02 M and Merthiolate® 0.1 mg/l (1997 Sigma catalogue), were suspended the cells of *Saccharomyces cerevisiae* obtained as described above; the mixture was maintained under gentle stirring for a period of 8 hours, at a temperature of 4-8°C. Then a further 73.5 g of CaCl₂ • 2H₂O were added to the reaction medium, so as to bring the calcium concentration up to 0.04 M. The mixture was then kept under agitation in the conditions referred to above, checking every 4 hours the calcium concentration using spectrophotometric techniques, and gradually adding further quantities of CaCl₂ until the concentration of the mineral in the medium was stabilized. Altogether, the mixture was kept under agitation for a period of 72 hours.

At the end of the intracellular introduction of the mineral, the suspension was centrifuged at 10,000 r.p.m. for 15 minutes. The micro-organic cells were then collected and washed with a sterile hypotonic solution containing CaCl₂ 0.02 M, not containing Merthiolate®, with the aim of eliminating possible residue of the antibacterial agent. After centrifugation, the cells were re-suspended in a small volume of sterile hypotonic solution (approximately 4-5 l).

### BIOLOGICAL ACTIVITY

### (i) Analysis of growth curves, food consumption and serum concentration of minerals in animals treated with the inactivated micro-organisms containing calcium according to the present invention.

Four groups of 30 rabbits in the growth step, weighing 0.8-1 kg each, belonging to both sexes, underwent different food treatments for a period of 90 days, according to the following scheme:
Group 1: 15 males and 15 females, with free access to food consisting of ordinary feed (controls);
Group 2: 15 males and 15 females, with free access to food consisting of the feed referred to above, mixed with 5 g of CaCl₂ • 2H₂O per kg of feed;
Group 3: 15 males and 15 females, with free access to food consisting of the same feed as above, enriched with inactivated cells of *Saccharomyces cerevisiae* containing Ca, prepared according to Example 1, in doses such that the quantity of Ca administered was equivalent to that administered to Group 2, corresponding to 1.805 g Ca²⁺ per kg of feed, determined using spectrophotometric techniques;
Group 4: 15 males and 15 females, with free access to food consisting of feed poor in Ca content.

The mineral Ca was chosen on account of its influence on bone constitution, as well as on the neuromuscular and cardiovascular systems, i.e., physiological mechanisms directly involved in the growth process.

The general state of the animals under examination was controlled every day, and every 7th day the body weight trend and food consumption was evaluated. At the end of the treatment described above, the animals were sacrificed for the anatomical and pathological control of the state of the gastro-intestinal mucous membrane, the main organs and systems (in particular the skeletal and muscular systems), and no significant alteration was detected.

Table 1 gives the percentage differences in body weight and food consumption, evaluated at the end of the treatment period, on the day before the animals were sacrificed, for the 4 groups of animals tested.

**Table 1**

| **Animals** | **% variation with respect to the controls** | |
|---|---|---|
| | **Body weight** | **Food consumption** |
| Group 1 | ---- | ---- |
| Group 2 | +4.2 | ---- |
| Group 3 | +14.6 | +2.0 |
| Group 4 | -8.7 | -4.5 |

The results given above show a significant increase in body weight in the animals treated with the inactivated micro-organisms containing Ca, according to the present invention, with respect to the control animals, in the presence of a non-significant variation in food consumption. These data indicate a marked assimilation and integration of the minerals.

On the eve of sacrifice, blood was taken from the 4 groups of animals treated for determination of the serum concentration of the mineral administered. The determination of the concentration of calcium ion was made using a spectrophotometric method, measuring the complex with cresolphthalein, according to the procedure described by A.J. Pesce *et al*. (*Methods in Clinical Chemistry*, Page 1003, 1987, Mosby Publ.).

Table 2 gives the percentage variations of Ca serum concentrations with respect to the controls.

**Table 2**

| **Animals** | **Ca % variation with respect to the controls** |
|---|---|
| Group 1 | ---- |
| Group 2 | +3.4 |
| Group 3 | +9.5 |
| Group 4 | -10.5 |

Behavioural analysis showed a marked improvement in the "performance" of the animals treated with the micro-organisms according to the present invention as compared to the controls.

In addition, the anatomical and pathological examination following on sacrifice did not detect any significant signs at the level of the intestinal mucous membrane. The skeletal and muscular systems of the animals treated with the micro-organisms according to the invention revealed a compactness and consistency significantly higher than the controls and than the animals treated with CaCl₂, and markedly better than in the case of the animals with a diet poor in Ca content.

### (ii) Analysis of Fe serum concentration in animals treated with the inactivated micro-organisms according to the present invention

The following tests were performed on 4 groups of 12 male Wistar rats, with body weight of 150 g, which underwent different food treatments, according to the following scheme:
Group 1: for a period of 60 days, free access to food consisting of ordinary feed (controls);
Group 2: for a period of 60 days, free access to food consisting of feed poor in Fe content;
Group 3: for the first 30 days, free access to food consisting of feed poor in Fe; for the subsequent 30 days, free access to food consisting of feed mixed with ferrous sulphate, in a concentration of 3 mg per kg of feed;
Group 4: for the first 30 days, free access to food consisting of feed poor in Fe content; for the subsequent 30 days, free access to food consisting of feed enriched with inactivated cells of *Azotobacter vinelandii* containing Ca according to the present invention, in concentrations such as to administer 3 mg of ferrous sulphate per kg of feed.

On the 30th day from the beginning of treatment, and subsequently every 10 days, the Fe serum levels were evaluated in the various groups of animals treated, using the spectrophotometric method with bathophenanthroline, as described by A.J. Pesce *et al*. (*Methods in Clinical Chemistry*, Pages 1258-1261, 1987, Mosby Publ.).

Table 3 gives the percentage variations, with respect to the controls which had received an ordinary diet, of the Fe serum concentration in the animals belonging to the groups referred to above, after the time intervals specified.

**Table 3**

| **Animals** | **Fe % variation with respect to the controls** | | | |
|---|---|---|---|---|
| | 30 days | 40 days | 50 days | 60 days |
| | from start of treatment | | | |
| Group 1 | ---- | ---- | ---- | ---- |
| Group 2 | -17.2 | -20.2 | -23.5 | -28.0 |
| Group 3 | -17.2 | -14.0 | -7.1 | +5.3 |
| Group 4 | -17.2 | -2.0 | +5.5 | +12.8 |

The results tabulated above witness to the fact that the micro-organisms according to the present invention are able to accelerate significantly the correction of a state of deficit of minerals induced by diets poor in such minerals. This effect is considerably more marked than that brought about by the direct administration of the mineral itself in the form of salt.

### (iii) Influence of the micro-organisms of the invention on the prevention of experimental infections

The following tests were conducted on 4 groups of 20 male Swiss mice, weighing 20±2 g, which were treated in the 20 days preceding induction of the experimental infection, according to the following scheme:
Group 1: free access to food consisting of ordinary feed (controls);
Group 2: free access to food consisting of feed mixed with 100 µg of zinc sulphate per kg of feed;
Group 3: free access to food consisting of feed enriched with *Saccharomyces cerevisiae* cells in a concentration of 20 g per kg of feed;
Group 4: free access to food consisting of feed enriched with *Saccharomyces cerevisiae* cells containing the complex of Zn with alginate of molecular weight 2,500-5,000 daltons according to the present invention, in concentrations such as to administer 100 µg of Zn ion per kg of feed.

After 20 days' feeding in the conditions described above, the animals were experimentally infected via intravenous injection of 4 • 10³ cells of *Salmonella* *typhimurium* (strain C5), according to the method described by B. Bizzini *et al*. (FEMS, *Microbiol. Immunol*., 64:155-168, 1990).

On the 5th day of infection, the number of animals that survived was checked. Table 4 gives the number and percentage variations of the animals which survived as compared to the control group.

**Table 4**

| **Animals** | **Number of animals that survived** | **% variation of animals survived** |
|---|---|---|
| Group 1 | 8 | ---- |
| Group 2 | 8 | 0 |
| Group 3 | 12 | +20 |
| Group 4 | 14 | +30 |

The results reported above witness to the fact that the treatment with cells of *Saccharomyces cerevisiae* leads to an increase in defences against experimental intestinal infections, as already pointed out previously. However, the simultaneous administration of the micro-organisms of the invention enriched in minerals is able to carry out a notable synergistic activity in this direction.

### (iv) Influence of the micro-organisms according to the invention containing Pt in association with cyclophosphamide on the growth of Lewis's solid tumour in rats

The following tests were conducted on 3 groups of 20 mice of the strain C 57 BL/6, weighing approximately 20 g, which were treated according to the following scheme:
Group 1: no treatment (controls);
Group 2: intraperitoneal administration of 10 mg/kg/day of cyclophosphamide;
Group 3: intraperitoneal administration of 10 mg/kg/day of cyclophosphamide associated to the simultaneous oral administration of inactivated cells of *Saccharomyces cerevisiae* containing *cis*-platinum complexed to alginate of molecular weight of 2,500-5,000 daltons according to the present invention, in such concentrations as to administer 2.5 mg of Pt ion/kg/day.

At the beginning of the test, each group of animals received grafts of Lewis's tumour via intramuscular injection of 10⁴ tumour cells per animal. Finally, the time necessary for obtaining 100% mortality with respect to the controls was determined, as reported in Table 5 below.

**Table 5**

| **Animals** | **Time (days) for 100% mortality** |
|---|---|
| Group 1 | 35 |
| Group 2 | 41 |
| Group 3 | 44 |

The results indicated above witness to the fact that the treatment with cyclophosphamide antagonizes the rate of development of Lewis's solid tumour, as is already widely known in the state of the art. The simultaneous association of cyclophosphamide with inactivated cells of *Saccharomyces cerevisiae* containing *cis*-platinum is able to increase to a higher degree the level of resistance of the animals treated.

## Claims

1. Process for producing inactivated micro-organisms containing one or more minerals in the form of complexes with polysaccharides, which comprises the following steps:
1) extraction of the endocellular mass from the cells of a suitable micro-organism by means of hyperosmotic treatment, followed by separation of the endocellular mass from the cell walls by centrifugation or filtration;
2) optional inactivation of the micro-organism obtained in step (1) by chemical or physical means, leaving the external membrane of the micro-organism unaltered;
3) intracellular insertion of said mineral complexes into the inactivated micro-organism obtained in step (1) or (2) by means of a hypo-osmotic treatment with a hypotonic suspension or solution comprising said mineral complexes with polysaccharides.

2. Process according to Claim 1, **characterized in that** said minerals are selected from the group consisting of Cu, Zn, Fe, Se, Cr, Mn, Ca, Mg, Mo, Au, Pt, Cd, V, Li, and K.

3. Process according to Claim 1, **characterized in that** said polysaccharides are chosen from the group consisting of alginates, xanthans, mannans, dermatan sulphates, chondroitin sulphates, dextrans and fragments thereof.

4. Process according to Claim 3, **characterized in that** said polysaccharides are depolymerized to obtain oligosaccharides of molecular weight of between 2,500 and 5,000 daltons.

5. Process according to Claim 1, **characterized in that** said micro-organism is not a producer of polysaccharides, and serves as a vehicling agent for said minerals.

6. Process according to claim 5, **characterized in that** said micro-organism is *Saccharomyces cerevisiae*

7. Process according to Claim 1, **characterized in that** said micro-organism is a producer of one or more polysaccharides, selected from the group consisting of alginates, xanthans, mannans, dermatan sulphates, chondroitin sulphates, and dextrans.

8. Process according to Claim 7, **characterized in that** said polysaccharides are alginates and the said micro-organism is *Azotobacter vinelandii* or *Pseudomonas aeruginosa.*

9. Process according to Claim 7, **characterized in that** said polysaccharides are xanthans and the said micro-organism is *Xanthomonas campestris*.

10. Process according to Claim 7, **characterized in that** said polysaccharides are phosphomannans and the said micro-organism is *Hansenula capsulata*.

11. Process according to Claim 1, **characterized in that**, before step (1), said micro-organisms are made to grow by fermentation, possibly recovering the polysaccharides produced during said fermentation.

12. Process according to Claim 1, **characterized in that**, during step (1), said hyperosmotic treatment consists in suspending the said micro-organism in a hypertonic solution containing:
- NaCl in concentrations of over 0.2 M;
- sodium citrate in concentrations of over 0.05 M;
- an appropriate antibacterial agent, in suitable concentrations;
and in keeping the said suspension under agitation at a temperature ranging between 2 and 8°C for a period of between 2 hours and 4 days.

13. Process according to Claim 1, **characterized in that**, in step (2), said chemical inactivation is obtained by treating the micro-organisms in an aqueous solution of a suitable disinfectant substance, at a concentration of 0.05-0.2 mg/l, for a period of 4-72 hours.

14. Process according to Claim 1, **characterized in that**, in step (2), said physical inactivation is obtained by exposure to UV rays or by heating to 55-65°C.

15. Process according to Claim 1, **characterized in that**, in step (3), the inactivated micro-organisms obtained in step (1) or (2) are suspended in a hypotonic aqueous solution containing said minerals in the form of complexes with polysaccharides, for a period of time of between 8 hours and 4 days, at a temperature ranging between 2 and 8°C.

16. Process according to Claim 15, **characterized in that** said hypotonic aqueous solution further contains an appropriate antibacterial agent.

17. Inactivated micro-organism containing minerals in the form of complexes with polysaccharides, obtainable as described in any of the claims from 1 to 16.

18. Food composition comprising an effective quantity of the micro-organisms according to claim 17, in association with appropriate foods, excipients and/or diluents.

19. Pharmaceutical composition comprising an effective quantity of the micro-organism according to claim 17.

20. Use of the inactivated micro-organisms according to claim 17, for the preparation of pharmaceuticals or of veterinary food.

## Patentansprüche

1. Verfahren zur Herstellung von inaktivierten Mikroorganismen enthaltend ein Mineral oder mehrere Mineralien in Form von Komplexen zusammen mit Polysacchariden, welches die folgenden Schritte umfasst:
1) Extraktion endozellulärer Masse aus Zellen von geeigneten Mikroorganismen durch hyperosmotische Behandlung, gefolgt von Abtrennung der endozellulären Masse von den Zellwänden mithilfe von Zentrifugation oder Filtration;
2) optional Inaktivierung von in Schritt (1) erhaltenen Mikroorganismen durch chemische oder physikalische Methoden, wobei die externe Membran der Mikroorganismen unverändert bleibt;
3) intrazelluläre Insertion von genannten Mineralkomplexen in die inaktivierten in Schritt (1) oder (2) erhaltenen Mikroorganismen durch hypoosmotische Behandlung mit einer hypotonischen Suspension oder Lösung beinhaltend genannte Mineralkomplexe mit Polysacchariden.

2. Verfahren gemäß Anspruch 1, **dadurch gekennzeichnet, dass** die genannten Mineralien ausgewählt werden aus der Gruppe bestehend aus Cu, Zn, Fe, Se, Cr, Mn, Ca, Mg, Mo, Au, Pt, Cd, V, U und K.

3. Verfahren gemäß Anspruch 1, **dadurch gekennzeichnet, dass** die genannten Polysaccharide aus der Gruppe ausgewählt werden bestehend aus Alginaten, Xanthanen, Mannanen, Dermatansulfaten, Chondroitinsulfaten, Dextranen und Fragmenten davon.

4. Verfahren gemäß Anspruch 3, **dadurch gekennzeichnet, dass** die genannten Polysaccharide depolymerisiert werden, um Oligosaccharide mit einem Molekulargewicht zwischen 2.500 und 5.000 Dalton zu erhalten.

5. Verfahren gemäß Anspruch 1, **dadurch gekennzeichnet, dass** der genannte Mikroorganismus keine Polysaccharide herstellt und als Trägeragenz für die genannten Mineralien dient

6. Verfahren gemäß Anspruch 5, **dadurch gekennzeichnet, dass** es sich bei dem genannten Mikroorganismus um *Saccharomyces cerevisiae* handelt.

7. Verfahren gemäß Anspruch 1, **dadurch gekennzeichnet, dass** der genannte Mikroorganismus ein Polysaccharid oder mehrere Polysaccharide herstellt, welche aus der Gruppe ausgewählt werden bestehend aus Alginaten, Xanthanen, Mannanen, Dermatansulfaten, Chondroitinsulfaten und Dextranen.

8. Verfahren gemäß Anspruch 7, **dadurch gekennzeichnet, dass** es sich bei den genannten Polysacchariden um Alginate und bei dem genannten Mikroorganismus um *Azotobacter vinelandii* oder *Pseudomonas aeruginosa* handelt.

9. Verfahren gemäß Anspruch 7, **dadurch gekennzeichnet, dass** es sich bei den genannten Polysacchariden um Xanthane und bei dem genannten Mikroorganismus um *Xanthomonas campestris* handelt.

10. Verfahren gemäß Anspruch 7, **dadurch gekennzeichnet, dass** es sich bei den genannten Polysacchariden um Phosphomannane und bei dem genannten Mikroorganismus um *Hansenula capsulata* handelt.

11. Verfahren gemäß Anspruch 1, **dadurch gekennzeichnet, dass** die genannten Mikroorganismen vor dem Schritt (1) durch Fermentation angezogen werden und möglicherweise die während der genannten Fermentation produzierten Polysaccharide gewonnen werden.

12. Verfahren gemäß Anspruch 1, **dadurch gekennzeichnet, dass** die hyperosmotische Behandlung während des Schritts (1) darin besteht, den genannten Mikroorganismus in einer hypertonischen Lösung zu suspendieren, welche enthält:
- NaCl in Konzentrationen von über 0,2 M;
- Natriumcitrat in Konzentrationen von über 0,05 M;
- ein entsprechendes antibakterielles Agens in geeigneten Konzentrationen;
und unter Rühren der genannten Suspension bei einer Temperatur im Bereich von 2 bis 8°C über einen Zeitraum von 2 Stunden bis zu 4 Tagen.

13. Verfahren gemäß Anspruch 1, **dadurch gekennzeichnet, dass** die in Schritt (2) genannte chemische Inaktivierung erreicht wird durch Behandlung der Mikroorganismen in einer wässrigen Lösung eines geeigneten Desinfektionsmittels bei einer Konzentration von 0,05-0,2 mg/l, über einen Zeitraum von 4-72 Stunden.

14. Verfahren gemäß Anspruch 1, **dadurch gekennzeichnet, dass** die in Schritt (2) genannte physikalische Inaktivierung erreicht wird durch Exposition gegenüber UV-Strahlung oder durch Erhitzen auf 55-65°C.

15. Verfahren gemäß Anspruch 1, **dadurch gekennzeichnet, dass** in Schritt (3) die inaktivierten Mikroorganismen aus Schritt (1) oder (2) in einer hypotonischen wässrigen, die genannten Mineralien in Form von Komplexen mit Polysacchariden enthaltenden Lösung über einen Zeitraum von 8 Stunden bis zu 4 Tagen in einem Temperaturbereich zwischen 2 und 8°C suspendiert werden.

16. Verfahren gemäß Anspruch 15, **dadurch gekennzeichnet, dass** die genannte hypotonische wässrige Lösung im Weiteren ein geeignetes antibakterielles Agens enthält.

17. Inaktivierte Mikroorganismen enthaltend Mineralien in der Form von Komplexen mit Polysacchariden, die wie in einem der Ansprüche von 1-16 gewonnen werden können.

18. Nahrungsmittelzusammensetzung umfassend eine wirksame Menge an Mikroorganismen gemäß Anspruch 17 in Verbindung mit geeigneten Nahrungsmitteln, Trägersubstanzen und/oder Verdünnungsmitteln.

19. Pharmazeutische Zusammensetzung umfassend eine wirksame Menge an Mikroorganismen gemäß Anspruch 17.

20. Verwendung der inaktivierten Mikroorganismen gemäß Anspruch 17 zur Herstellung von Pharmazeutika oder Tierfutter.

## Revendications

1. Procédé de production de microorganismes inactivés contenant un ou plusieurs minéraux sous la forme de complexes avec des polysaccharides, qui comprend les étapes suivantes:
1) extraction de la masse endocellulaire des cellules d'un microorganisme approprié au moyen d'un traitement hyperosmotique, avec ensuite séparation de la masse endocellulaire des parois des cellules par centrifugation ou filtration;
2) inactivation facultative du microorganisme obtenu à l'étape (1) par un moyen chimique ou physique, laissant la membrane externe du microorganisme non modifiée.
3) insertion intracellulaire desdits complexes minéraux dans le microorganisme inactivé obtenu à l'étape (1) ou (2) au moyen d'un traitement hypo-osmotique avec une suspension ou solution hypotonique comprenant lesdits complexes minéraux avec des polysaccharides.

2. Procédé selon la revendication 1, **caractérisé en ce que** lesdits minéraux sont sélectionnés dans le groupe consistant en Cu, Zn, Fe, Se, Cr, Mn, Cn, Mg, Mo, Au, Pt, Cd, V, Li, et K.

3. Procédé selon la revendication 1, **caractérisé en ce que** lesdits polysaccharides sont choisis dans le groupe consistant en alginates, xanthanes, mannanes, sulfates de dermatan, sulfates de chondroïtine, dextranes et leurs fragments.

4. Procédé selon la revendication 3, **caractérisé en ce que** lesdits polysaccharides sont dépolymérisés pour obtenir des oligosaccharides d'un poids moléculaire compris entre 2500 et 5000 daltons.

5. Procédé selon la revendication 1, **caractérisé en ce que** ledit microorganisme n'est pas un producteur de polysaccharides, et sert d'agent véhiculant pour lesdits minéraux.

6. Procédé selon la revendication 5, **caractérisé en ce que** ledit microorganisme est *Saccharomyces cerevisiae.*

7. Procédé selon la revendication 1, **caractérisé en ce que** ledit microorganisme est un producteur d'un ou plusieurs polysaccharides, sélectionnés dans le groupe consistant en alginates, xanthanes, mannanes, sulfates de dermatan, sulfates de chondroitine, et dextranes.

8. Procédé selon la revendication 7, **caractérisé en ce que** lesdits polysaccharides sont des alginates et ledit microorganisme est *Azotobacter vinelandii* ou *Pseudomonas aeruginosa*.

9. Procédé selon la revendication 7, **caractérisé en ce que** lesdits polysaccharides sont des xanthanes et ledit microorganisme est *Xanthomonas campestris*.

10. Procédé selon la revendication 7, **caractérisé en ce que** lesdits polysaccharides sont des phosphomannanes et ledit microorganisme est *Hansenula capsulata*.

11. Procédé selon la revendication 1, **caractérisé en ce qu'**avant l'étape (1), lesdits microorganismes sont forcés à croître par fermentation, en récupérant éventuellement les polysaccharides produits ladite fermentation.

12. Procédé selon la revendication 1, **caractérisé en ce que**, pendant l'étape (1), ledit traitement hyperosmotique consiste à mettre ledit microorganisme en suspension dans une solution hypertonique contenant:
- NaCl à des concentrations de plus de 0,2 M;
- citrate de sodium à des concentrations de plus de 0,05 M;
- un agent antibactérien approprié, à des concentrations appropriées;
et à maintenir ladite suspension sous agitation à une température comprise entre 2 et 8°C pendant une période comprise entre 2 heures et 4 jours.

13. Procédé selon la revendication 1, **caractérisé en ce qu'**à l'étape (2), ladite inactivation chimique est obtenue par traitement des microorganismes dans une solution aqueuse d'une substance désinfectante appropriée, à une concentration de 0,05-0,2 mg/l, pendant une période de 4-72 heures.

14. Procédé selon la revendication 1, **caractérisé en ce qu'**à l'étape (2), ladite inactivation physique est obtenue par exposition à des rayons UV ou par chauffage à 55-65°C.

15. Procédé selon la revendication 1, **caractérisé en ce qu'**à l'étape (3), les microorganismes inactivés obtenus à l'étape (1) ou (2) sont mis en suspension dans une solution aqueuse hypotonique contenant lesdits minéraux sous la forme de complexes avec des polysaccharides, pendant une période de temps comprise entre 8 heures et 4 jours, à une température comprise entre 2 et 8°C.

16. Procédé selon la revendication 15, **caractérisé en ce que** ladite solution aqueuse hypotonique contient de plus un agent antibactérien approprié.

17. Microorganisme inactivé contenant des minéraux sous la forme de complexes avec des polysaccharides, que l'on peut obtenir comme décrit dans l'une quelconque des revendications 1 à 16.

18. Composition alimentaire comprenant une quantité efficace de microorganismes selon la revendication 17, en association avec des aliments, excipients et/ou diluants appropriés.

19. Composition pharmaceutique comprenant une quantité efficace du microorganisme selon la revendication 17.

20. Utilisation des microorganismes inactivés selon la revendication 17, pour la préparation de produits pharmaceutiques ou d'aliments vétérinaires.
